Europäisches Patentamt

(19) European Patent Office

Office européen des brevets

(11) Numéro de publication : **0 089 921**

**B1**

(12) **FASCICULE DE BREVET EUROPÉEN**

(45) Date de publication du fascicule du brevet :
29.07.87

(51) Int. Cl.⁴ : **A 61 F 9/00**

(21) Numéro de dépôt : 83810090.7

(22) Date de dépôt : 04.03.83

(54) **Tête optique d'une installation pour l'observation et le traitement par rayonnement laser de l'oeil.**

(30) Priorité : 11.03.82 CH 1495/82
16.04.82 FR 8206716

(43) Date de publication de la demande :
28.09.83 Bulletin 83/39

(45) Mention de la délivrance du brevet :
29.07.87 Bulletin 87/31

(84) Etats contractants désignés :
CH DE FR GB IT LI NL

(56) Documents cités :
EP-A- 0 030 210
CH-A- 453 571
GB-A- 965 131
GB-A- 2 074 343
US-A- 3 315 680

(73) Titulaire : **LASAG AG**
**Bernstrasse 11**
**CH-3600 Thun (CH)**

(72) Inventeur : **Roussel, Philippe**
**Anemonenweg 1**
**NL-2241 XH Wassenaar (NL)**

(74) Mandataire : **Gresset, Jean et al**
**ICB Ingénieurs Conseils en Brevets SA Faubourg du Lac 6**
**CH-2501 Bienne (CH)**

## Description

La présente invention concerne les installations pour l'observation et le traitement par rayonnement laser de l'œil, et elle se rapporte plus particulièrement à la tête optique d'une telle installation.

Un certain nombre d'affections de l'œil, comme par exemple différents types de glaucomes ou de cataractes, ou les troubles résultant de la formation dans l'œil de membranes ou de filaments d'origines diverses, peuvent actuellement être traitées par l'application dans certaines régions de la chambre antérieure ou postérieure de l'œil d'un rayonnement laser de forte densité.

A cet effet, on utilise des installations de traitement ophtalmologique, généralement complexes, qui combinent un générateur laser de puissance, fixe, pour produire le faisceau laser de traitement, et une tête optique mobile susceptible d'être approchée de l'œil du patient, et permettant notamment d'amener le faisceau de traitement sur la zone à traiter.

Une installation de ce type est décrite par exemple dans la demande de brevet EP-A-0 230 210 (Fig. 2) au nom de la demanderesse. Le générateur laser de puissance qu'elle utilise est un laser Nd-YAG pouvant délivrer en mode Q-Switch des impulsions très courtes et de très forte intensité. La tête optique de l'installation comporte quant à elle un dispositif d'éclairage produisant un faisceau d'éclairage de la zone de l'œil à traiter, un dispositif d'observation de cette zone, et un générateur d'un faisceau laser de marquage.

Le faisceau de marquage est produit par exemple par un laser He-Ne et est donc constitué d'un rayonnement visible. Sa fonction est de matérialiser de façon précise le point de focalisation du faisceau de traitement, avant la libération de l'impulsion, ainsi que l'enveloppe de ce même faisceau. Cette dernière caractéristique permet à l'opérateur de l'installation de s'assurer que le faisceau de traitement n'atteindra pas au passage une zone différente de celle qui doit être traitée. Le faisceau de marquage est obtenu à la sortie du générateur au moyen d'un mécanisme à prismes tournants qui divisent le rayonnement laser produit en deux faisceaux élémentaires qui tournent autour du faisceau de traitement de manière à matérialiser son enveloppe. La détection de la rencontre fortuite de l'un de ces faisceaux élémentaires avec un obstacle est encore améliorée par un dispositif modulateur interrompant alternativement l'un ou l'autre faisceau.

Le faisceau d'éclairage est combiné avec ce faisceau de marquage, et ces deux faisceaux sont focalisés sur le point de l'œil à observer et à traiter par une première lentille.

Le faisceau de traitement est focalisé par une deuxième lentille et dirigé ensuite sur le point de l'œil à traiter par un miroir semi-transparent disposé sur le trajet des faisceaux de marquage et d'éclairage.

Les deux lentilles ci-dessus sont couplées mécaniquement de manière que leurs foyers coïncident en permanence.

Le miroir semi-transparent mentionné ci-dessus est réfléchissant pour la longueur d'onde du faisceau de traitement et transparent pour les longueurs d'onde des faisceaux de marquage et d'éclairage.

Le faisceau d'observation traverse également ce miroir semi-transparent et la lentille de focalisation des faisceaux de marquage et d'éclairage. Un filtre optique atténuant la longueur d'onde du faisceau de traitement est interposé sur le trajet de ce faisceau d'observation avant que celui-ci n'atteigne le dispositif d'observation.

La tête optique susmentionnée est donc relativement complexe à cause du nombre assez élevé d'éléments optiques tels que prismes, miroirs et lentilles qui sont nécessaires pour réaliser les diverses fonctions décrites. Cette complexité est agravée par le fait que les faisceaux de marquage et d'éclairage, d'une part, et le faisceau de traitement, d'autre part, sont focalisés par des lentilles distinctes liées mécaniquement l'une à l'autre. En outre, le simple filtre disposé sur le chemin optique du faisceau d'observation n'est pas suffisant pour garantir absolument qu'aucun rayonnement provenant indirectement du faisceau de traitement ne pourra atteindre le dispositif d'observation et blesser les yeux du chirurgien conduisant l'opération.

La demande de brevet GB-A-2 074 343 décrit une tête optique du genre mentionné ci-dessus qui ne présente pas ce dernier danger grâce à la présence d'un écran mobile qui interrompt complètement le faisceau d'observation lorsque le faisceau de traitement est émis. Mais, évidemment, cet écran empêche aussi le chirurgien de contrôler directement les effets du faisceau de traitement sur l'œil de son patient.

La tête optique décrite dans ce document GB-A-2 074 343 comporte en outre un générateur d'un faisceau de marquage, et ce faisceau de marquage et le faisceau de traitement sont focalisés sur le point à traiter par une lentille unique. Cette dernière caractéristique simplifie considérablement la construction de la tête optique. Mais le faisceau de marquage est simplement colinéaire au faisceau de traitement. Il ne permet donc pas de vérifier avec une sécurité absolue si le faisceau de traitement, lorsqu'il sera émis, ne va pas rencontrer et éventuellement endommager une partie de l'œil autre que celle qui doit être traitée.

Le but de la présente invention est de proposer une tête optique d'une installation pour l'observation et le traitement de l'œil par rayonnement laser du genre de celles qui ont été décrites ci-dessus, dans laquelle le faisceau de marquage forme une enveloppe visible du faisceau de traitement, comme dans la demande EP-A-0 030 210 mentionnée ci-dessus, dans laquelle ces deux faisceaux sont focalisés sur le point de l'œil à

traiter par une lentille unique comme dans la demande GB-A-2 074 343 également mentionnée ci-dessus, mais avec laquelle le chirurgien conduisant l'opération peut contrôler directement les effets du faisceau de traitement sur l'œil de son patient sans risquer que ses yeux ne soient blessés par une partie du faisceau de traitement qui serait réfléchie par l'un quelconque des éléments disposés sur son trajet.

Ce but est atteint par la tête optique décrite par la revendication 1 de la présente invention, qui est munie, selon un aspect essentiel de l'invention, d'un miroir disposé en amont de la lentille de focalisation et qui assure simultanément la réflexion sur cette lentille des faisceaux de traitement et de marquage, et la transmission du faisceau d'observation.

Les faisceaux de marquage et l'observation étant constitués de rayonnement visible, à l'inverse du faisceau de traitement ce miroir met à profit la localisation du faisceau de marquage formant l'enveloppe du faisceau de traitement. A cet effet, il comporte une portion centrale qui assure le passage du faisceau d'observation et la réflexion du faisceau de traitement, et une portion annulaire que vient frapper le faisceau de marquage, et qui est traitée de manière à le réfléchir également.

L'invention sera bien comprise à la lecture de la description suivante, faite en liaison avec les dessins joints, parmi lesquels :

la figure 1 est une vue schématique de la tête optique selon l'invention ; et

la figure 2 est une vue du miroir particulier mis en œuvre dans la tête optique de la figure 1.

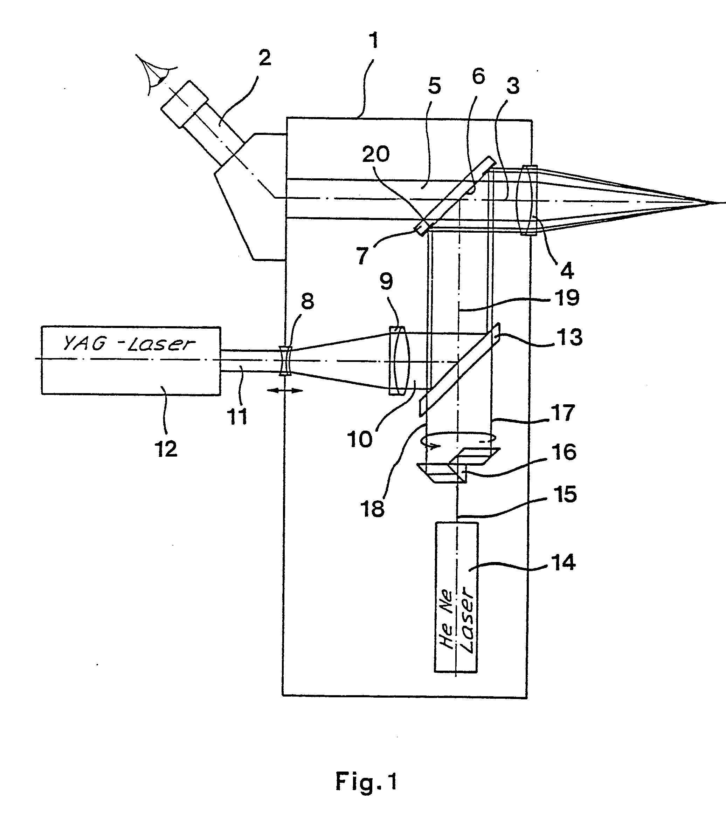

Comme on le voit au mieu dans la figure 1, la tête optique 1 comprend notamment des moyens d'observation 2 sur l'axe optique 3 desquels est disposée la lentille de focalisation 4. Les moyens d'observation 2 peuvent être constitués par exemple par un ensemble binoculaire stéréoscopique classique. Le faisceau d'observation 5 qui amène l'image du foyer F de la lentille 4 aux moyens d'observation 2 traverse directement la portion centrale 6 d'un premier miroir 7 placé devant la lentille 4. Cette partie centrale ayant la forme d'un cercle est formée d'une surface présentant un coefficient de transmission maximal dans le visible.

Par ailleurs, la tête optique 1 comprend également une lentille de défocalisation 8 réglable en position et une lentille convergente 9 définissant un faisceau de traitement 10 à partir du rayonnement cohérent 11 produit à l'extérieur de la tête 1 par un générateur 12 du type laser Nd-YAG dont la longueur d'onde de fonctionnement est de l'ordre de 1.06 μm. Les lentilles 8 et 9 constituent ainsi un agrandisseur de faisceau cohérent 11. Un deuxième miroir 13 présentant un coefficient de réflexion maximal pour cette longueur d'onde envoie alors le faisceau de traitement 10 sur la portion centrale du premier miroir 7, dont la surface présente cette même caractéristique. Le faisceau de traitement 10 est ainsi dirigé sur la lentille de focalisation 10 coaxialement au faisceau d'observation 5.

Enfin, la tête optique 1 comprend encore un générateur laser 14, par exemple du type He-Ne, émettant dans le visible un rayonnement cohérent 15. Un dispositif de séparation et de mise en rotation 16 est associé au générateur 14 pour former un faisceau de marquage constitué par deux parties ou rayons élémentaires 17 et 18 tournant autour d'un axe 19 qui coïncide avec celui du faisceau de traitement 10 en amont du premier miroir 7. L'écartement des rayons 17 et 18 est très légèrement supérieur au diamètre du faisceau de traitement 10, de manière à délimiter l'enveloppe extérieure de ce dernier. A sa sortie du dispositif 16 de séparation et de mise en rotation, le faisceau de marquage 17, 18 traverse le second miroir 13, qui présente un coefficient de transmission maximal dans le visible, et vient frapper le premier miroir 7. Une portion annulaire 20 de ce dernier, entourant sa portion centrale 6 est prévue pour réfléchir le faisceau de marquage 17, 18 vers la lentille de focalisation 4. A cet effet, cette portion annulaire 20 porte un revêtement réfléchissant dans le visible, ou du moins pour la longueur d'onde du faisceau de marquage, par exemple à base d'aluminium, dont le diamètre intérieur est supérieur à celui du faisceau d'observation 5 pour ne pas l'intercepter, et dont le diamètre extérieur est supérieur à l'écartement des rayons 17 et 18 du faisceau de marquage.

Les moyens d'observation 2, les générateurs lasers 12 et 14, le dispositif de séparation et de mise en rotation 16, la lentille de focalisation 4, ainsi que le fonctionnement de l'installation dans son ensemble ont été exposés de façon très détaillée dans la demande de brevet européen précitée, et il n'est donc pas utile d'en reprendre ici la description.

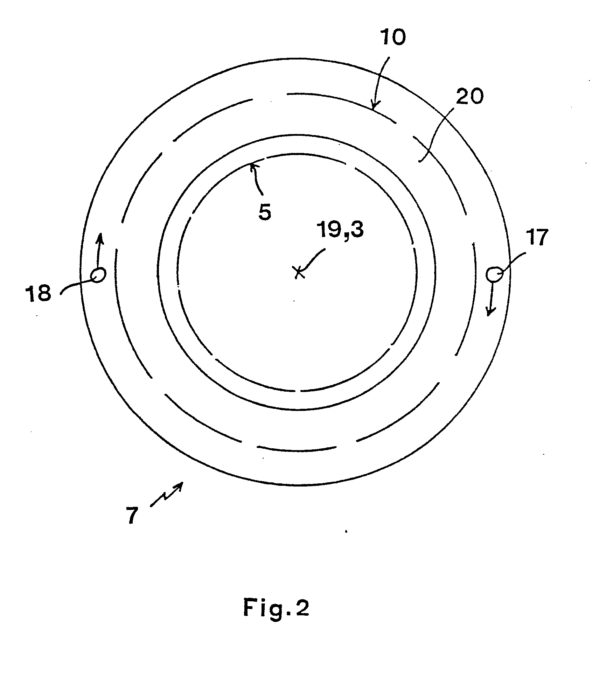

Dans la figure 2, on a représenté le premier miroir 7 de la tête optique, ainsi que la trace de l'intersection des faisceaux d'observation 5, de traitement 10, de marquage 17 et 18 avec sa portion centrale 6 et sa portion extérieure annulaire 20. Ce miroir est de préférence un miroir dichroïque, qui présente dans sa position centrale un coefficient de réflexion maximal pour la longueur d'onde du laser Nd-YAG, de l'ordre de 95 % à 98 % par exemple, et un coefficient de transmission maximal dans le visible, de l'ordre de 80 % à 90 % par exemple.

**Revendications**

1. Tête optique d'une installation pour l'observation et le traitement par rayonnement laser d'un œil, comprenant des moyens (8, 9) pour produire un faisceau (10) de rayonnement laser de traitement, des moyens (14, 16) pour produire un faisceau de rayonnement laser de marquage (17, 18) formant une enveloppe visible du faisceau (10) de traitement, des moyens d'observation (2) de la zone de l'œil à traiter destinés à recevoir un faisceau d'observation (5), une lentille de focalisation (4) dont l'axe coïncide avec l'axe optique (3) des moyens d'observation (2) et qui assure la focalisation sur la zone de l'œil à traiter des faisceaux de traitement (10) et de marquage (17, 18), et un premier miroir (7) interceptant les faisceaux de traitement (10) et de marquage (17, 18) et disposé entre la lentille de focalisation (4) et les moyens d'observation (2) pour réfléchir vers cette lentille de focalisation (4) les faisceaux de traitement (10) et de marquage (17, 18), caractérisée en ce que le premier miroir (7) comprend une portion extérieure (20) interceptant uniquement le faisceau de marquage (17, 18) et présentant un coefficient de réflexion maximal pour la longueur d'onde du faisceau de marquage (17, 18) et une portion centrale (6) interceptant uniquement le faisceau de traitement (10) et présentant un coefficient de réflexion maximal pour la longueur d'onde du faisceau de traitement (10) et un coefficient de transmission maximal pour la longueur d'onde du faisceau de marquage (17, 18), ladite portion centrale (6) assurant ainsi la réflexion du faisceau de traitement (10) vers la zone à traiter et la transmission vers les moyens d'observation (2) dudit faisceau d'observation (5) et empêchant en outre la transmission du faisceau de traitement (10) vers les moyens d'observation (2) après réflexion sur la zone à traiter.

2. Tête optique selon la revendication 1, caractérisée en ce que la portion centrale (6) du premier miroir (7) a la forme d'un cercle centré sur l'axe optique (3) des moyens d'observation (2) et la portion extérieure (20) du premier miroir (7) a la forme d'un anneau entourant ledit cercle.

3. Tête optique selon l'une des revendications précédentes, caractérisée en ce que les moyens (14, 16) pour produire le faisceau de marquage (17, 18) comprennent un générateur (14) d'un faisceau laser visible (15) coaxial au faisceau de traitement (10), en ce que les moyens (8, 9) pour produire le faisceau de traitement (10) comportent une lentille d'entrée (8) pour permettre à un faisceau laser (11) produit à l'extérieur de la tête optique (1) et ayant la longueur d'onde du faisceau de traitement (10) d'entrer dans la tête optique (1), et en ce que ladite tête optique (1) comporte en outre un deuxième miroir (13) présentant un coefficient de réflexion minimum pour la longueur d'onde du faisceau laser visible (15) et maximum pour la longueur d'onde du faisceau laser (11) produit à l'extérieur de la tête optique (1), ledit deuxième miroir (13) interceptant le faisceau laser visible (15) et le faisceau laser (11) produit à l'extérieur de la tête optique (1) pour produire le faisceau de marquage (17, 18) par transmission du faisceau laser visible (11) et le faisceau de traitement (10) par réflexion du faisceau laser (15) produit à l'extérieur de la tête optique (1).

4. Tête optique selon la revendication 3, caractérisée en ce que la lentille d'entrée (8) est une lentille divergente, et en ce que les moyens (8, 9) pour produire le faisceau de traitement (10) comportent en outre une lentille convergente (9) disposée entre la lentille d'entrée (8) et le deuxième miroir (13), ces deux lentilles (8, 9) constituant un agrandisseur pour le faisceau laser (11) produit à l'extérieur de la tête optique (1).

5. Tête optique selon l'une des revendications 3 ou 4, caractérisée en ce que les moyens pour produire le faisceau de marquage (17, 18) comportent des moyens (16) pour séparer le faisceau laser visible (15) en deux faisceaux séparés (17, 18) tournant symétriquement par rapport à l'axe (19) des faisceaux de traitement (10) et de marquage (17, 18).

## Claims

1. Optical head of an installation for observation and treatment of an eye by laser radiation, comprising means (8, 9) for producing a treatment beam (10) of laser radiation, means (14, 16) for producing a marking beam (17, 18) of laser radiation forming a visible envelope of the treatment beam (10), observation means (2) for the zone of the eye to be treated intended to receive an observation beam (5), a focussing lens (4) the axis of which coincides with the optical axis (3) of the observation means (2) and which assures focussing of the treatment beam (10) and marking beam (17, 18) on the zone of the eye to be treated, and a first mirror (7) intercepting the treatment beam (10) and marking beam (17, 18) and placed between the focussing lens (4) and the observation means (2) for reflecting the treatment beam (10) and the marking beam (17, 18) toward such focussing lens (4) characterized in that the first mirror (7) comprises an outer portion (20) intercepting solely the making beam (17, 18) and having a maximum coefficient of reflection for the wavelength of the marking beam (17, 18) and a central portion (6) intercepting solely the treatment beam (10) and having a maximum coefficient of reflection for the wavelength of the treatment beam (10) and a maximum transmission coefficient for the wavelength of the marking beam (17, 18) said central portion (6) thus assuring reflection of the treatment beam (10) toward the zone to be treated and transmission toward the observation means (2) of said observation beam (5) and furthermore preventing transmission of the treatment beam (10) toward the observation means (2) after reflection on the zone to be treated.

2. Optical head according to claim 1 characterized in that the central portion (6) of the first mirror (7) has the form of a circle centered on the optical axis (3) of the observation means (2) and the outer portion (20) of the first mirror (7) has the form of a ring surrounding said circle.

3. Optical head according to either of the preceding claims characterized in that the means (14, 16) for producing the marking beam (17, 18) comprise a generator (14) of a visible laser beam (15) coaxial to the treatment beam (10), in that the means (8, 9) for producing the treatment beam (10) include an entry lens (8) to permit a laser beam (11) produced outside the optical head (1) and having the wavelength of the treatment beam to enter the optical head (1), and in that said optical head (1) furthermore includes a second mirror (13) having a coefficient of reflection which is minimum for the wavelength of the visible laser beam (15) and maximum for the wavelength of the laser beam (11) produced outside the optical head (1), said second mirror (13) intercepting the visible laser beam (15) and the laser beam (11) produced outside the optical head (1) for producing the marking beam (17, 18) by transmission of the visible laser beam (15) and the treatment beam (10) by reflection of the laser beam (11) produced outside the optical head (1).

4. Optical head according to claim 3 characterized in that the entry lens (8) is a divergent lens and in that the means (8, 9) for producing the treatment beam (10) furthermore include a convergent lens (9) placed between the entry lens (8) and the second mirror (13), these two lenses (8, 9) constituting an enlarger for the laser beam (11) produced outside the optical head.

5. Optical head according to either of claims 3 or 4 characterized in that the means for producing the marking beam (17, 18) include means (16) for separating the visible laser beam (15) into two separate beams (17, 18) turning symmetrically relative to the axis (19) of the treatment beam (10) and the marking beam (17, 18).

**Patentansprüche**

1. Optischer Kopf einer Anlage für die Untersuchung und Behandlung durch Laserstrahlung eines Auges mit Mitteln (8, 9) zum Erzeugen eines Behandlungslaserstrahlenbündels (10), Mitteln (14, 16) zum Erzeugen eines Markierlaserstrahlenbündels (17, 18), das eine sichtbare Hülle des Behandlungsbündels (10) bildet, Mitteln zum Beobachten (2) der zu behandelnden Zone des Auges, bestimmt zum Aufnehmen eines Beobachtungsbündels (5), einer Fokalisationslinse (4), deren Achse mit der optischen Achse (3) der Beobachtungsmittel (2) zusammenfällt und die die Fokalisation des Behandlungs- und des Markierbündels auf die zu behandelnde Augenzone gewährleistet, und einem ersten Spiegel (7), der im Strahlengang des Behandlungs- (10) und Markier- (17, 18) bündels sowie zwischen der Fokalisationslinse (4) und den Beobachtungsmitteln (2) angeordnet ist, um in Richtung der Fokalisationslinse (4) das Behandlungs- (10) und Markier- (17, 18) bündel zu reflektieren dadurch gekennzeichnet, daß der erste Spiegel (7) einen Außenabschnitt (20) aufweist, der im Strahlengang nur des Markierbündels (17, 18) angeordnet ist und einen maximalen Reflexionskoeffizienten aufweist für die Wellenlänge des Markierbündels (17, 18) und einen zentralen Abschnitt (6), der im Strahlengang nur des Behandlungsbündels (10) angeordnet ist und einen maximalen Reflexionskoeffizienten für die Wellenlänge des Behandlungsbündels (10) aufweist sowie einen maximalen Transmissionskoeffizienten für die Wellenlänge des Markierbündels (17, 18), wobei der genannte zentrale Abschnitt (6) auf diese Weise die Reflexion des Behandlungsbündels (10) in Richtung der zu behandelnden Zone und die Transmission in Richtung der Beobachtungsmittel (2) des Beobachtungsbündels (5) gewährleistet und im übrigen die Transmission des Behandlungsbündels (10) in Richtung der Beobachtungsmittel (2) nach Reflexion auf die zu behandelnde Zone unterbindet.

2. Optischer Kopf nach Anspruch 1, dadurch gekennzeichnet, daß der zentrale Abschnitt (6) des ersten Spiegels (7) die Form eines auf die optische Achse (3) der Beobachtungsmittel (2) zentrierten Kreises aufweist und der äußere Abschnitt (20) des Spiegels (7) die Form eines diesen Kreis umgebenden Ringes besitzt.

3. Optischer Kopf nach einem der vorangehenden Ansprüche, dadurch gekennzeichnet, daß die Mittel (14, 16) zum Erzeugen des Markierbündels (17, 18) einen Generator (14) für einen sichtbaren Laserstrahl (15), koaxial mit dem Behandlungsbündel (10) umfassen, daß die Mittel (8, 9) zum Erzeugen des Behandlungsbündel (10) eine Eingangslinse (8) umfassen, um einen außerhalb des optischen Kopfes (1) erzeugten Laserstrahl (11) mit der Wellenlänge des Behandlungsbündels (10) den Eintritt in den optischen Kopf (1) zu ermöglichen, und daß der genannte optische Kopf (1) ferner einen zweiten Spiegel (13) umfaßt mit einem minimalen Reflexionskoeffizienten für die Wellenlänge des sichtbaren Laserbündels (15) und maximalem Reflexionskoeffizienten für die Wellenlänge des außerhalb des optischen Kopfes (1) erzeugten Laserbündels (11), welcher zweite Spiegel (13) im Strahlengang des sichtbaren Laserbündels (15) und des außerhalb des optischen Kopfes (1) erzeugten Laserbündels (11) angeordnet ist zum Erzeugen des Markierbündels (17, 18) durch Transmission des sichtbaren Laserbündels (15) und des Behandlungsbündels (10) durch Reflexion des außerhalb des optischen Kopfes (1) erzeugten Laserbündels (11).

4. Optischer Kopf nach Anspruch 3, dadurch gekennzeichnet, daß die Eingangslinse (8) eine divergierende Linse ist und daß die Mittel (8, 9) zum Erzeugen des Behandlungsbündels (10) ferner eine konvergierende Linse (9) umfassen, zwischen der Eingangslinse (8) und dem zweiten Spiegel (13) angeordnet, wobei diese beiden Linsen (8, 9) eine Vergrößerungseinrichtung für den außerhalb des optischen Kopfes (1) erzeugten Laserstrahl bilden.

5. Optischer Kopf nach einem der Ansprüche 3 oder 4, dadurch gekennzeichnet, daß die Mittel zum Erzeugen des Markierbündels (17, 18) Mittel (16) umfassen zum Aufteilen des sichtbaren Laserbündels (15) in zwei getrennte Strahlen (17, 18), die symmetrisch bezüglich der Achse (19) des Behandlungsbündels (10) und Markierbündel (17, 18) umlaufen.

# 0 089 921

**Fig.1**

Fig.2